# EUROPEAN PATENT SPECIFICATION

(11) **EP 0 937 067 B1**
(45) Date of publication and mention of the grant of the patent: **09.07.2003**
(21) Application number: 97946494.8
(22) Date of filing: 04.11.1997
(51) Int. Cl.: C07D 403/06, A61K 31/55

(54) **1-(3-AMINOINDAZOL-5-YL)-3-PHENYLMETHYL-CYCLIC UREAS USEFUL AS HIV PROTEASE INHIBITORS**
1-(3-AMINOINDAZOL-5-YL)-3-PHENYLMETHYL CYCLISCHE HARNSTOFFE UND IHRE VERWENDUNG ALS HIV PROTEASE INHIBITOREN
UREES 1-(3-AMINOINDAZOL-5-YL)-3-PHENYLMETHYL-CYCLIQUES UTILES EN TANT QU'INHIBITEURS DE PROTEASE DE VIH

(30) Priority: 08.11.1996 US 747104; 08.11.1996 US 29745 P
(43) Date of publication of application: 25.08.1999
(73) Proprietor: Bristol-Myers Squibb Pharma Company, Princeton, New Jersey 08543-4000 (US)
(72) Inventor: RODGERS, James David, Landenberg, PA 19350 (US); KALTENBACH, Robert Frank III, Wilmington, DE 19808 (US)
(74) Representative: Beacham, Annabel Rose
(86) International application number: US9720035
(87) International publication number: WO98020008

(56) References cited:
- WO-A-94/19329
- US-A- 5 532 357
- US-A- 5 610 294

## Description

This invention relates generally to 1-(3-aminoindazol-5-yl)-3-phenylmethyl-cyclic ureas which are useful as inhibitors of HIV protease, pharmaceutical compositions and diagnostic kits comprising the same, and the use of the same in the manufacture of a medicament for the treatment of HIV infection.

Two distinct retroviruses, human immunodeficiency virus (HIV) type-1 (HIV-1) or type-2 (HIV-2), have been etiologically linked to the immunosuppressive disease, acquired immunodeficiency syndrome (AIDS). HIV seropositive individuals are initially asymptomatic but typically develop AIDS related complex (ARC) followed by AIDS. Affected individuals exhibit severe immunosuppression which predisposes them to debilitating and ultimately fatal opportunistic infections.

The disease AIDS is the end result of an HIV-1 or HIV-2 virus following its own complex life cycle. The virion life cycle begins with the virion attaching itself to the host human T-4 lymphocyte immune cell through the bonding of a glycoprotein on the surface of the virion's protective coat with the CD4 glycoprotein on the lymphocyte cell. Once attached, the virion sheds its glycoprotein coat, penetrates into the membrane of the host cell, and uncoats its RNA. The virion enzyme, reverse transcriptase, directs the process of transcribing the RNA into single-stranded DNA. The viral RNA is degraded and a second DNA strand is created. The now double-stranded DNA is integrated into the human cell's genes and those genes are used for cell reproduction.

At this point, the human cell carries out its reproductive process by using its own RNA polymerase to transcribe the integrated DNA into viral RNA. The viral RNA is translated into the precursor *gag-pol* fusion polyprotein. The polyprotein is then cleaved by the HIV protease enzyme to yield the mature viral proteins. Thus, HIV protease in responsible for regulating a cascade of cleavage events that lead to the virus particle's maturing into a virus that is capable of full infectivity.

The typical human immune system response, killing the invading virion, is taxed because a large portion of the virion's life cycle is spent in a latent state within the immune cell. In addition, viral reverse transcriptase, the enzyme used in making a new virion particle, is not very specific, and causes transcription mistakes that result in continually changed glycoproteins on the surface of the viral protective coat. This lack of specificity decreases the immune system's effectiveness because antibodies specifically produced against one glycoprotein may be useless against another, hence reducing the number of antibodies available to fight the virus. The virus continues to reproduce while the immune response system continues to weaken. Eventually, the HIV largely holds free reign over the body's immune system, allowing opportunistic infections to set in and without the administration of antiviral agents, immunomodulators, or both, death may result.

There are at least three critical points in the virus's life cycle which have been identified as possible targets for antiviral drugs: (1) the initial attachment of the virion to the T-4 lymphocyte or macrophage site, (2) the transcription of viral RNA to viral DNA (reverse transcriptase, RT), and (3) the assemblage of the new virus particle during reproduction (e.g., HIV aspartic acid protease or HIV protease).

The genomes of retroviruses encode a protease that is responsible for the proteolytic processing of one or more polyprotein precursors such as the *pol* and *gag* gene products. See Wellink, *Arch. Virol.* 98 1 (1988). Retroviral proteases most commonly process the *gag* precursor into the core proteins, and also process the *pol* precursor into reverse transcriptase and retroviral protease.

The correct processing of the precursor polyproteins by the retroviral protease is necessary for the assembly of the infectious virions. It has been shown that *in vitro* mutagenesis that produces protease-defective virus leads to the production of immature core forms which lack infectivity. See Crawford et al., *J. Virol.* 53 899 (1985); Katoh et al., *Virology* 145 280 (1985). Therefore, retroviral protease inhibition provides an attractive target for antiviral therapy. See Mitsuya, *Nature* 325 775 (1987).

The ability to inhibit a viral protease provides a method for blocking viral replication and therefore a treatment for viral diseases, such as AIDS, that may have fewer side effects, be more efficacious, and be less prone to drug resistance when compared to current treatments. As a result, three HIV protease inhibitors, Roche's saquinavir, Abbott's ritonavir, and Merck's indinavir, are currently being marketed and a number of potential protease inhibitors are in clinical trials, e.g., Vertex's VX-478, Agouron's nelfinavir, Japan Energy's KNI-272, and Ciba-Geigy's CGP 61755.

As evidenced by the protease inhibitors presently marketed and in clinical trials, a wide variety of compounds have been studied as potential HIV protease inhibitors. One core, cyclic ureas, has received significant attention. For example, in PCT Application Number WO94/19329, Lam et al generically describe cyclic ureas of the formula: 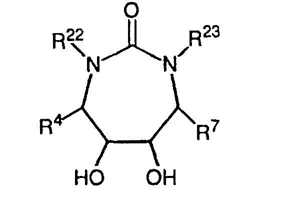 and methods of preparing these ureas. Though the present compounds fall within the description of Lam et al, they are not specifically disclosed therein.

US 5,532,357, Rodgers et al, relates to methods for the preparation of unsymmetrically substituted cyclic ureas which are useful as HIV protease inhibitors.

Even with the current success of protease inhibitors, it has been found that HIV patients can become resistant to a single protease inhibitor. Thus, it is desirable to develop additional protease inhibitors to further combat HIV infection.

Accordingly, one object of the present invention is to provide novel protease inhibitors.

It is another object of the present invention to provide pharmaceutical compositions with protease inhibiting activity comprising a pharmaceutically acceptable carrier and a therapeutically effective amount of at least one of the compounds of the present invention or a pharmaceutically acceptable salt or prodrug form thereof.

It is another object of the present invention to provide a novel use of a compound in the manufacture of a medicament for treating HIV infection.

It is another object of the present invention to provide a therapeutically effective combination of (a) one of the compounds of the present invention and (b) one or more compounds selected form the group consisting of HIV reverse transcriptase inhibitors and HIV protease inhibitors for use in treating HIV infection.

These and other objects, which will become apparent during the following detailed description, have been achieved by the inventors' discovery that the compound of formula II: 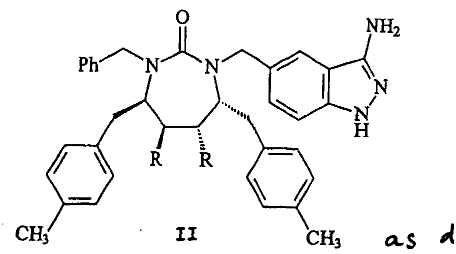 as defined below , are effective protease inhibitors.

Thus, in a first embodiment, the present invention provides a novel compound of formula II: 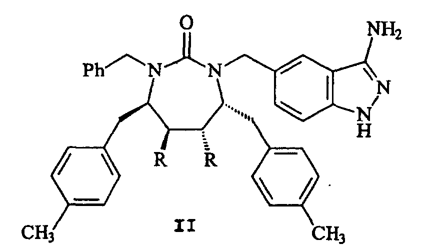 wherein each R is OH or a pharmaceutically acceptable salt thereof,
or a compound of formula II wherein both R groups taken together form a moiety selected from the group -O-,-OCH₂SCH₂O-, -OC(=O)O-, -OCH₂O-, -OC(=S)O-, -OC(=O)C(=O)O-,-OC(CH₃)₂O-, -OC((CH₂)₃NH₂)(CH₃)O-, -OC(OCH₃)(CH₂CH₂CH₃)O-, or-OS (=O)O-.

In a second embodiment, the present invention provides a novel pharmaceutical composition comprising a pharmaceutically acceptable carrier and a therapeutically effective amount of a compound of the present invention.

In a third embodiment, the present invention provides a compound of formula II for use in treating HIV infection.

In a fourth embodiment, the present invention provides a novel use of a compound of the present invention in the manufacture of a medicament for use in treating HIV infection.

In a fifth embodiment, the present invention provides a novel combination of:
(a) a compound of the present invention; and,
(b) at least one compound selected from the group consisting of HIV reverse transcriptase inhibitors and HIV protease inhibitors for use in treating HIV infection.

In another preferred embodiment, the reverse transcriptase inhibitor is a nucleoside reverse transcriptase inhibitor.

In another more preferred embodiment, the nucleoside reverse transcriptase inhibitor is selected from AZT, 3TC, ddI, ddC, and d4T and the protease inhibitor is selected from saquinavir, ritonavir, indinavir, VX-478, nelfinavir, KNI-272, CGP-61755, and U-103017.

In an even more preferred embodiment, the nucleoside reverse transcriptase inhibitor is selected from AZT and 3TC and the protease inhibitor is selected from saquinavir, ritonavir, and indinavir.

In a still further preferred ebodiment, the nucleoside reverse transcriptase inhibitor is AZT.

In another still further preferred embodiment, the protease inhibitor is indinavir.

In a sixth embodiment, the present invention provides a pharmaceutical kit useful for the treatment of HIV infection, which comprises a therapeutically effective amount of:
(a) a compound of the invention; and,
(b) at least one compound selected from the group consisting of HIV reverse transcriptase inhibitors and HIV protease inhibitors, in one or more sterile containers.

In another preferred embodiment, the compound is of formula I.

As used herein, the following terms and expressions have the indicated meanings. It will be appreciated that the compounds of the present invention contain an asymmetrically substituted carbon atom, and may be isolated in optically active or racemic forms. It is well known in the art how to prepare optically active forms, such as by resolution of racemic forms or by synthesis, from optically active starting materials. All chiral, diastereomeric, racemic forms and all geometric isomeric forms of a structure are intended, unless the specific stereochemistry or isomer form is specifically indicated.

As used herein, "HIV reverse transcriptase inhibitor" is intended to refer to both nucleoside and non-nucleoside inhibitors of HIV reverse transcriptase (RT). Examples of nucleoside RT inhibitors include, but are not limited to, AZT, ddC, ddI, d4T, and 3TC. Examples of non-nucleoside RT inhibitors include, but are no limited to, viviradine (Pharmacia and Upjohn U90152S), TIBO derivatives, BI-RG-587, nevirapine, L-697,661, LY 73497, and Ro 18,893 (Roche).

As used herein, "HIV protease inhibitor" is intended to refer to compounds which inhibit HIV protease. Examples include, but are not limited, saquinavir (Roche, Ro31-8959), ritonavir (Abbott, ABT-538), indinavir (Merck, MK-639), VX-478 (Vertex/Glaxo Wellcome), nelfinavir (Agouron, AG-1343), KNI-272 (Japan Energy), CGP-61755 (Ciba-Geigy), and U-103017 (Pharmacia and Upjohn). Additional examples include the cyclic protease inhibitors disclosed in WO93/07128, WO 94/19329, WO 94/22840, and PCT Application Number US96/03426.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in *Remington's Pharmaceutical* *Sciences,* 17th ed., Mack Publishing Company, Easton, PA, 1985, p. 1418, the disclosure of which is hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

"Prodrugs" are carriers which release the active parent drug according to formula II *in vivo* when such prodrug is administered to a mammalian subject. Prodrugs of a compound of the present invention are prepared by modifying functional groups present in the compound in such a way that the modifications are cleaved, either in routine manipulation or *in vivo*, to the parent compound. Prodrugs of the invention are compounds wherein the two R groups of formula II join to form an epoxide; -OCH₂SCH₂O-; -OC(=O)O-; -OCH₂O-; -OC(=S)O-; -OC(=O)C(=O)O-; -OC(CH₃)₂O-; -OC((CH₂)₃NH₂)(CH₃)O-; -OC(OCH₃)(CH₂CH₂CH₃)O-; or -OS(=O)O-.

"Stable compound" and "stable structure" are meant to indicate a compound that is sufficiently robust to survive isolation to a useful degree of purity from a reaction mixture, and formulation into an efficacious therapeutic agent. Only stable compounds are contempleted by the present invention.

"Therapeutically effective amount" is intended to include an amount of a compound of the present invention or an amount of the combination of compounds claimed effective to inhibit HIV infection or treat the symptoms of HIV infection in a host. The combination of compounds is preferably a synergistic combination. Synergy, as described for example by Chou and Talalay, Adv. Enzyme Regul. 22:27-55 (1984), occurs when the effect (in this case, inhibition of HIV replication) of the compounds when administered in combination is greater than the additive effect of the compounds when administered alone as a single agent. In general, a synergistic effect is most clearly demonstrated at suboptimal concentrations of the compounds. Synergy can be in terms of lower cytotoxicity, increased antiviral effect, or some other beneficial effect of the combination compared with the individual components.

Other features of the invention will become apparent in the course of the following descriptions of exemplary embodiments which are given for illustration of the invention and are not intended to be limiting thereof.

### Examples

Abbreviations used in the Examples are defined as follows: "°C" for degrees Celsius, "d" for doublet, "dd" for doublet of doublets, "eq" for equivalent or equivalents, "g" for gram or grams, "mg" for milligram or milligrams, "mL" for milliliter or milliliters, "H" for hydrogen or hydrogens, "hr" for hour or hours, "m" for multiplet, "M" for molar, "min" for minute or minutes, "MHz" for megahertz, "MS" for mass spectroscopy, "nmr" or "NMR" for nuclear magnetic resonance spectroscopy, "t" for triplet, and "TLC" for thin layer chromatography.

### EXAMPLE 1

### Preparation of (4R,5S,6S,7R)-Hexahydro-1-[5-(3-aminoindazole)methyl]-5,6-dihydroxy-4,7-bis[(4-methylphenyl)methyl]-3-phenylmethyl-2H-1,3-diazapin-2-one (II).

**PART A:** (2R,3S,4S,5R)-2,5-Bis(2,2-dimethylhydrazo)-1,6-bis(4-methylphenyl)-3,4-O-isopropylidenehexanediol (10).

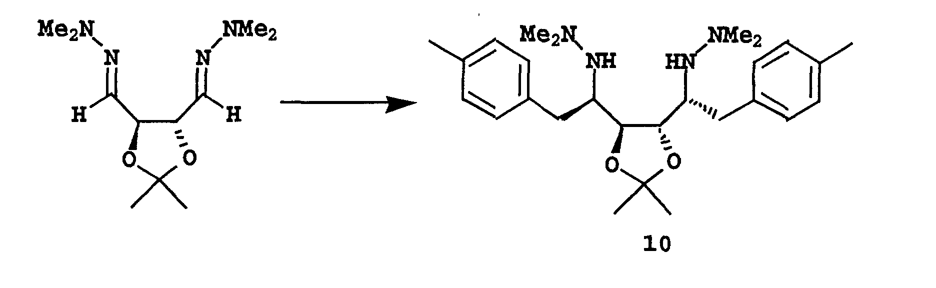

The starting hydrazone can be prepared by the method of Rossano et al (see Formula 3a on page 4968 of *Tetr. Lett.* **1995,** *36(28)*, 4967-70.

To p-xylene (57 mL, 464 mmol) at 15°C was added sec-butyllithium (95 mL, 123 mmol, 1.3 M in cyclohexane) dropwise over 5 min. The solution was cooled to -15°C and THF (30 mL) was added dropwise. After stirring lh, the hydrazone (10.2 g, 42 mmol) in THF (30 mL) was added dropwise. The reaction mixture was stirred 20 min. and was warmed to 0°C. The solution was carefully quenched with water and was extracted with EtOAc. The combined organic layers were extracted with 1N HCl and the combined aqueous fractions were made strongly basic with 50% aqueous NaOH. The resulting mixture was extracted with EtOAc, washed with brine and dried (MgSO₄). The solvent was removed under reduced pressure to give the bis-hydrazine **10** as an oil (19.25 g, 99%): ¹H NMR (CDCl₃) δ 7.08 (s, 8 H), 4.09 (s, 2 H), 3.02 (t, J = 7.1 Hz, 2 H), 2.67 (m, 4 H), 2.31 (s, 6 H), 2.17 (s, 12 H), 1.43 (s, 6 H); IR (KBr) ν 2980, 2940, 1680, 1510, 1240 cm⁻¹; LRMS (ESI) m/z: 455 (M+H⁺, 6%), 228 (M+2H⁺, 100%); HRMS calcd. for C₂₇H₄₃N₄O₂ (M+H⁺) 455.3386; found 455.3393.

### PART B: (2R,3S,4S,5R)-2,5-Diamino-1,6-bis(4-methylphenyl)-3,4-0-isopropylidenehexanediol (11).

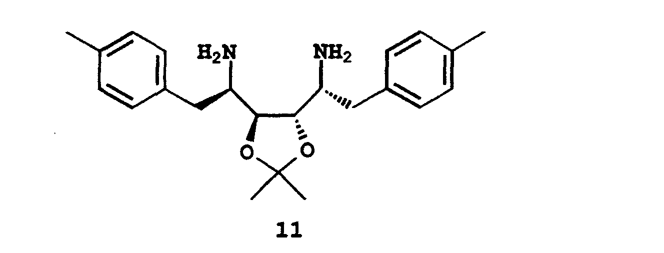

To a solution of bis-hydrazine **10** (18.64 g, 41 mmol) in methanol (150 mL) was added Raney nickel (20 g, 50% slurry). The suspension was charged with hydrogen (250 psi) and heated at 100°C for 16 h. The suspension was cooled, filtered through celite, and the solvent was removed under reduced pressure. Chromatography (silica gel, 10% methanol/CH₂Cl₂) gave the diamine **11** as an oil (12.49 g, 83%): ¹H NMR (CDCl₃) δ 7.08 (ab, J = 8.1 Hz, Δν = 15.2 Hz, 8 H), 4.01 (s, 2 H), 2.94 (m, 2 H), 2.77 (A of ABX, JAB = 13.4 Hz, JAX = 4.6 Hz, 2 H), 2.51 (B of ABX, JAB = 13.4 Hz, JBX = 9.7 Hz, 2 H), 2.32 (s, 6 H), 1.45 (s, 6 H); IR (KBr) ν 2980, 2920, 1510 cm⁻¹; LRMS (ESI) m/z: 369 (M+H⁺, 16%), 185 (M+2H⁺, 100%); HRMS calcd. for C₂₃H₃₃N₂O₂ (M+H⁺) 369.2542; found 369.2534.

### PART C: (4R,5S,6S,7R)-Hexahydro-5,6-O-isopropylidene-4,7-bis[(4-methylphenyl)methyl]-2H-1,3-diazapin-2-one (12).

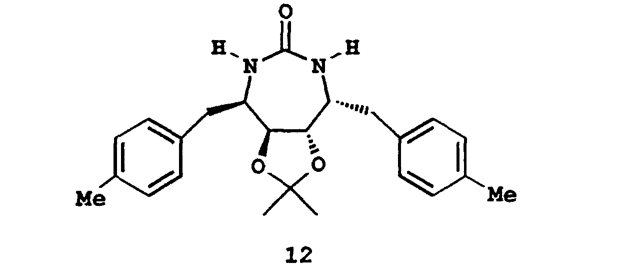

To a solution of diamine **11** (12.48 g, 33.9 mmol) in 1,1,2,2-tetrachloroethane (130 mL) was added 1,1'-carbonyldiimidazole (5.67 g, 35.0 mmol). After 10 min. the solution was added dropwise over 45 min. to 1,1,2,2-tetrachloroethane (600 mL) at reflux. The solution was cooled, washed with water, brine, and dried (MgSO₄). The solvent was removed under reduced pressure and the residue was chromatographed (silica gel, 33% ethyl acetate/hexane) followed by recrystalization (ethyl acetate/hexane) to give cyclic urea **12** as a white solid (6.18 g, 46%) : mp. 228-230°C; ¹H NMR (CDCl₃) δ 7.12 (br s, 8 H), 4.93 (d, J = 6.2 Hz, 2 H), 4.25 (s, 2 H), 3.50 (m, 2 H), 3.01 (app. d, J = 13.2 Hz, 2 H), 2.78 (app. t, J = 11.8 Hz, 2 H), 2.33 (s, 6 H), 1.54 (s, 6 H); IR (KBr) ν 3260, 2930, 1670, 1090 cm⁻¹; LRMS (ESI) m/z: 395 (M+H⁺, 100%); HRMS calcd. for C₂₄H₃₁N₂O₃ (M+H⁺) 395.2335; found 395.2333.

### PART D: (4R,5S,6S,7R)-Hexahydro-5,6-O-isopropylidene-4,7-bis[(4-methylphenyl)methyl]-1-phenylmethyl-2H-1,3-diazapin-2-one (13).

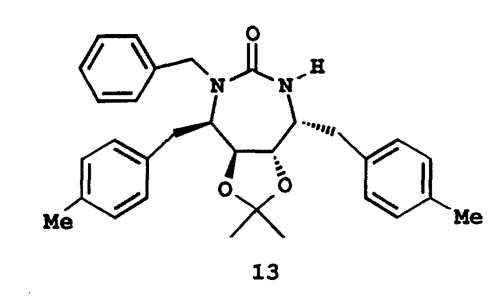

To a solution of cyclic urea **12** (3.0 g, 7.6 mmol) and benzyl bromide (1.59 g, 9.3 mmol) in THF (300 mL) at 0°C was added potassium *t*-butoxide (8.4 mL, 8.4 mmol, 1.0 M in THF) dropwise over 30 min. The solution was allowed to warm to rt and was stirred overnight. The reaction mixture was diluted with brine and was extracted with EtOAc. The combined organic layers were washed with brine and dried (MgSO₄). The solvent was removed under reduced pressure and the residue was chromatographed (silica gel, 25% ethyl acetate/hexane) to give cyclic urea **13** as a glass (2.57 g, 70%): ¹H NMR (CDCl₃) δ 7.28 (m, 3 H), 7.15 (m, 10 H), 5.12 (d, J = 14.6 Hz, 1 H), 4.88 (d, J = 6.6 Hz, 1 H), 4.23 (m, 1 H), 3.75 (m, 2 H), 3.47 (m, 1 H), 3.01 (m, 3 H), 2.86 (d, J = 14.6 Hz, 1 H), 2.66 (m, 1 H), 2.37 (s, 3 H), 2.34 (s, 3 H), 1.45 (s, 3 H), 1.40 (s, 3 H); IR (KBr) ν 2930, 1650, 1240, 1090 cm⁻¹; LRMS (ESI) m/z: 485 (M+H⁺, 100%); HRMS calcd. for C₃₁H₃₇N₂O₃ (M+H⁺) 485.2804; found 485.2789.

### PART E: (4R,5S,6S,7R)-Hexahydro-1-[(3-cyano-4-fluorophenyl)methyl]-5,6-O-isopropylidene-4,7-bis[(4-methylphenyl)methyl]-3-phenylmethyl-2H-1,3-diazapin-2-one (14).

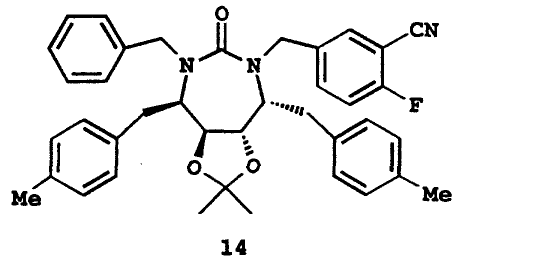

To a solution of cyclic urea **13** (2.30 g, 4.75 mmol) and 3-cyano-4-fluorobenzyl bromide (1.07 g, 5.0 mmol) in THF (200 mL) at 0°C was added potassium *t*-butoxide (4.8 mL, 4.8 mmol, 1.0 M in THF). The solution was warmed to rt and stirred overnight. The reaction mixture was diluted with brine and was extracted with EtOAc. The combined organic layers were washed with brine and dried (MgSO₄). The solvent was removed under reduced pressure and the residue was chromatographed (silica gel, 25% ethyl acetate/hexane) to give cyclic urea **14** as a glass (2.43 g, 82%): ¹H NMR (CDCl₃) δ7.33 (m, 5 H), 7.13 (d, J = 7.7 Hz, 4 H), 6.95 (m, 7 H), 4.89 (d, J = 14.3 Hz, 1 H), 4.46 (d, J = 14.3 Hz, 1 H), 3.97 (m, 1 H), 3.78 (m, 3 H), 3.65 (d, J = 14.3 Hz, 1 H), 3.07 (d, J = 14.3 Hz, 1 H), 2.83 (m, 4 H), 2.36 (s, 3 H), 2.34 (s, 3 H), 1.44 (s, 3 H), 1.38 (s, 3 H); IR (KBr) ν 2980, 2930, 2240, 1630, 1230 cm⁻¹; CIMS (NH₄) m/z: 635 (M+NH₄⁺, 100%); HRMS calcd. for C₃₉H₄₁N₃O₃F (M+H⁺) 618.3132; found 618.3119.

### PART F: (4R,5S,6S,7R)-Hexahydro-1-[5-(3-aminoindazole)methyl]-5,6-O-isopropylidene-4,7-bis[(4-methylphenyl)methyl]-3-phenylmethyl-2H-1,3-diazapin-2-one (15).

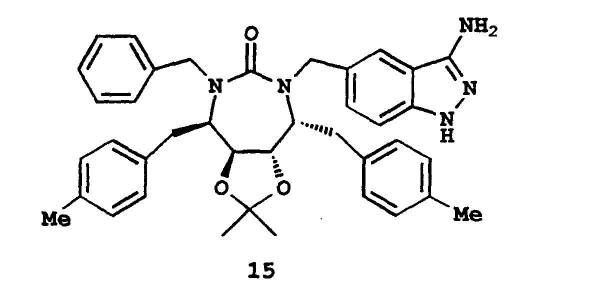

To a solution of cyclic urea **14** (2.40 g, 3.89 mmol) in n-butanol (40 mL) was added hydrazine monohydrate (19.8 g, 395 mmol) and the resulting solution was refluxed overnight. The reaction mixture was diluted with water and was extracted with EtOAc. The combined organic layers were washed with brine and dried (MgSO₄). The solvent was removed under reduced pressure and the residue was chromatographed (silica gel, 10% methanol/methylene chloride) to give aminoindazole **15** as a white solid (2.34 g, 95%): mp 120-124 °C; ¹H NMR (CDCl₃) δ 7.25 (m, 12 H), 6.97 (t, J = 7.7 Hz, 4 H), 4.98 (dd, J = 14.3, 2.0 Hz, 2 H), 3.80 (s, 2 H), 3.76 (m, 2 H), 3.27 (d, J = 14.3 Hz, 1 H), 3.09 (d, J = 14.3 Hz, 1 H), 2.86 (m, 4 H), 2.35 (s, 6 H), 1.32 (s, 6 H); IR (KBr) ν 3310, 2930, 1630, 1430, 1230 cm⁻¹; CIMS (NH₄) m/z: 630 (M+H⁺, 100%); HRMS calcd. for C₃₉H₄₄N₅O₃ (M+H⁺) 630.3444; found 630.3428.

### PART G: (4R,5S,6S,7R)-Hexahydro-1-[5-(3-aminoindazole)methyl]-5,6-dihydroxy-Preparation of 4,7-bis[(4-methylphenyl)methyl]-3-phenylmethyl-2H-1,3-diazapin-2-one (II).

Cyclic urea **15** (1.90 g, 3.02 mmol) was dissolved in 10% conc. HCl in methanol (40 mL). After 2 h, saturated aqueous Na₂CO₃ was added and the suspension was extracted with EtOAc. The combined organic layers were washed with brine and dried (MgSO₄). The solvent was removed under reduced pressure and the residue was chromatographed (silica gel, 10% methanol/methylene chloride) to give the title compound as a white solid (1.71 g, 96%): mp 142-146 °C; ¹H NMR (CD₃OD) δ 7.37 (s, 1 H) , 7.28 (m, 3 H), 7.22 (s, 2 H), 7.15 (m, 6 H), 6.94 (m, 4 H), 4.77 (d, J = 14.3 Hz, 1 H), 4.72 (d, J = 14.3 Hz, 1 H), 3.59 (m, 2 H), 3.51 (br s, 2 H), 3.11 (d, J = 14.3 Hz, 1 H), 2.93 (m, 5 H), 2.31 (br s, 6 H) ; IR (KBr) ν 3330, 2920, 1610, 1470, 1230 cm⁻¹; CIMS (NH₃) m/z: 590 (M+H⁺, 100%); HRMS calcd. for C₃₆H₄₀N₅O₃ (M+H⁺) 590.3131; found 590.3132; Anal. (C₃₆H₃₉N₅O₃) C, H, N.

### Utility

The compounds of formula II possess HIV protease inhibitory activity and are therefore useful as antiviral agents for the treatment of HIV infection and associated diseases. The compounds of formula II possess HIV protease inhibitory activity and are effective as inhibitors of HIV growth. The ability of the compounds of the present invention to inhibit viral growth or infectivity is demonstrated in standard assay of viral growth or infectivity, for example, using the assay described below.

The compounds of formula II of the present invention are also useful for the inhibition of HIV in an *ex vivo* sample containing HIV or expected to be exposed to HIV. Thus, the compounds of the present invention may be used to inhibit HIV present in a body fluid sample (for example, a serum or semen sample) which contains or is suspected to contain or be exposed to HIV.

The compounds provided by this invention are also useful as standard or reference compounds for use in tests or assays for determining the ability of an agent to inhibit viral clone replication and/or HIV protease, for example in a pharmaceutical research program. Thus, the compounds of the present invention may be used as a control or reference compound in such assays and as a quality control standard. The compounds of the present invention may be provided in a commercial kit or container for use as such standard or reference compound.

Since the compounds of the present invention exhibit specificity for HIV protease, the compounds of the present invention may also be useful as diagnostic reagents in diagnostic assays for the detection of HIV protease. Thus, inhibition of the protease activity in an assay (such as the assays described herein) by a compound of the present invention would be indicative of the presence of HIV protease and HIV virus.

As used herein "µg" denotes microgram, "mg" denotes milligram, "g" denotes gram, "µL" denotes microliter, "mL" denotes milliliter, "L" denotes liter, "nM" denotes nanomolar, "µM" denotes micromolar, "mM" denotes millimolar, "M" denotes molar and "nm" denotes nanometer. "Sigma" stands for the Sigma-Aldrich Corp. of St. Louis, MO.

### HIV RNA Assay

### DNA Plasmids and in vitro RNA transcripts:

Plasmid pDAB 72 containing both gag and pol sequences of BH10 (bp 113-1816) cloned into PTZ 19R was prepared according to Erickson-Viitanen et al. *AIDS Research and Human Retroviruses* **1989,** 5, 577. The plasmid was linearized with Bam HI prior to the generation of *in vitro* RNA transcripts using the Riboprobe Gemini system II kit (Promega) with T7 RNA polymerase. Synthesized RNA was purified by treatment with RNase free DNAse (Promega), phenol-chloroform extraction, and ethanol precipitation. RNA transcripts were dissolved in water, and stored at -70°C. The concentration of RNA was determined from the A₂₆₀.

### Probes:

Biotinylated capture probes were purified by HPLC after synthesis on an Applied Biosystems (Foster City, CA) DNA synthesizer by addition of biotin to the 5' terminal end of the oligonucleotide, using the biotin-phosphoramidite reagent of Cocuzza, *Tet. Lett.* **1989,** *30*, 6287. The gag biotinylated capture probe (5-biotin-CTAGCTCCCTGCTTGCCCATACTA 3') was complementary to nucleotides 889-912 of HXB2 and the pol biotinylated capture probe (5'-biotin -CCCTATCATTTTTGGTTTCCAT 3' ) was complementary to nucleotides 2374-2395 of HXB2. Alkaline phosphatase conjugated oligonucleotides used as reporter probes were prepared by Syngene (San Diego, CA.). The pol reporter probe (5' CTGTCTTACTTTGATAAAACCTC 3') was complementary to nucleotides 2403-2425 of HXB2. The gag reporter probe (5' CCCAGTATTTGTCTACAGCCTTCT 3') was complementary to nucleotides 950-973 of HXB2. All nucleotide positions are those of the GenBank Genetic Sequence Data Bank as accessed through the Genetics Computer Group Sequence Analysis Software Package (Devereau *Nucleic Acids Research* **1984,** *12,* 387). The reporter probes were prepared as 0.5 µM stocks in 2 x SSC (0.3 M NaCl, 0.03 M sodium citrate), 0.05 M Tris pH 8.8, 1 mg/mL BSA. The biotinylated capture probes were prepared as 100 µM stocks in water.

### Streptavidin coated plates:

Streptavidin coated plates were obtained from Du Pont Biotechnology Systems (Boston, MA).

### Cells and virus stocks:

MT-2 and MT-4 cells were maintained in RPMI 1640 supplemented with 5% fetal calf serum (FCS) for MT-2 cells or 10% FCS for MT-4 cells, 2 mM L-glutamine and 50 µg/mL gentamycin, all from Gibco. HIV-1 RF was propagated in MT-4 cells in the same medium. Virus stocks were prepared approximately 10 days after acute infection of MT-4 cells and stored as aliquots at -70°C. Infectious titers of HIV-1(RF) stocks were 1-3 x 10⁷ PFU (plaque forming units)/mL as measured by plaque assay on MT-2 cells (see below). Each aliquot of virus stock used for infection was thawed only once.

For evaluation of antiviral efficacy, cells to be infected were subcultured one day prior to infection. On the day of infection, cells were resuspended at 5 x 10⁵ cells/mL in RPMI 1640, 5% FCS for bulk infections or at 2 x 10⁶/mL in Dulbecco's modified Eagles medium with 5% FCS for infection in microtiter plates. Virus was added and culture continued for 3 days at 37°C.

### HIV RNA assay:

Cell lysates or purified RNA in 3 M or 5 M GED were mixed with 5 M GED and capture probe to a final guanidinium isothiocyanate concentration of 3 M and a final biotin oligonucleotide concentration of 30 nM. Hybridization was carried out in sealed U bottom 96 well tissue culture plates (Nunc or Costar) for 16-20 hours at 37°C. RNA hybridization reactions were diluted three-fold with deionized water to a final guanidinium isothiocyanate concentration of 1 M and aliquots (150 µL) were transferred to streptavidin coated microtiter plates wells. Binding of capture probe and capture probe-RNA hybrid to the immobilized streptavidin was allowed to proceed for 2 hours at room temperature, after which the plates were washed 6 times with DuPont ELISA plate wash buffer (phosphate buffered saline(PBS), 0.05% Tween 20.) A second hybridization of reporter probe to the immobilized complex of capture probe and hybridized target RNA was carried out in the washed streptavidin coated well by addition of 120 µl of a hybridization cocktail containing 4 X SSC, 0.66% Triton X 100, 6.66% deionized formamide, 1 mg/mL BSA and 5 nM reporter probe. After hybridization for one hour at 37°C, the plate was again washed 6 times.
Immobilized alkaline phosphatase activity was detected by addition of 100 µL of 0.2 mM 4-methylumbelliferyl phosphate (MUBP, JBL Scientific) in buffer δ(2.5 M diethanolamine pH 8.9 (JBL Scientific), 10 mM MgCl₂, 5 mM zinc acetate dihydrate and 5 mM N-hydroxyethyl-ethylene-diamine-triacetic acid). The plates were incubated at 37°C. Fluorescence at 450 nM was measured using a microplate fluorometer (Dynateck) exciting at 365 nM.

### Microplate based compound evaluation in HIV-1 infected MT-2 cells:

Compounds to be evaluated were dissolved in DMSO and diluted in culture medium to twice the highest concentration to be tested and a maximum DMSO concentration of 2%. Further three-fold serial dilutions of the compound in culture medium were performed directly in U bottom microtiter plates (Nunc). After compound dilution, MT-2 cells (50 µL) were added to a final concentration of 5 x 10⁵ per mL (1 x 10⁵ per well). Cells were incubated with compounds for 30 minutes at 37°C in a CO₂ incubator. For evaluation of antiviral potency, an appropriate dilution of HIV-1 (RF) virus stock (50 µL) was added to culture wells containing cells and dilutions of the test compounds. The final volume in each well was 200 µL. Eight wells per plate were left uninfected with 50 µL of medium added in place of virus, while eight wells were infected in the absence of any antiviral compound. For evaluation of compound toxicity, parallel plates were cultured without virus infection.

After 3 days of culture at 37°C in a humidified chamber inside a CO₂ incubator, all but 25 µL of medium/well was removed from the HIV infected plates. Thirty seven µL of 5 M GED containing biotinylated capture probe was added to the settled cells and remaining medium in each well to a final concentration of 3 M GED and 30 nM capture probe. Hybridization of the capture probe to HIV RNA in the cell lysate was carried out in the same microplate well used for virus culture by sealing the plate with a plate sealer (Costar), and incubating for 16-20 hrs in a 37°C incubator. Distilled water was then added to each well to dilute the hybridization reaction three-fold and 150 µL of this diluted mixture was transferred to a streptavidin coated microtiter plate. HIV RNA was quantitated as described above. A standard curve, prepared by adding known amounts of pDAB 72 *in vitro* RNA transcript to wells containing lysed uninfected cells, was run on each microtiter plate in order to determine the amount of viral RNA made during the infection.

In order to standardize the virus inoculum used in the evaluation of compounds for antiviral activity, dilutions of virus were selected which resulted in an IC₉₀ value (concentration of compound required to reduce the HIV RNA level by 90%) for dideoxycytidine (ddC) of 0.2 µg/mL. IC₉₀ values of other antiviral compounds, both more and less potent than ddC, were reproducible using several stocks of HIV-1 (RF) when this procedure was followed. This concentration of virus corresponded to ∼3 x 10⁵ PFU (measured by plaque assay on MT-2 cells) per assay well and typically produced approximately 75% of the maximum viral RNA level achievable at any virus inoculum. For the HIV RNA assay, IC₉₀ values were determined from the percent reduction of net signal (signal from infected cell samples minus signal from uninfected cell samples) in the RNA assay relative to the net signal from infected, untreated cells on the same culture plate (average of eight wells). Valid performance of individual infection and RNA assay tests was judged according to three criteria. It was required that the virus infection should result in an RNA assay signal equal to or greater than the signal generated from 2 ng of pDAB 72 *in vitro* RNA transcript. The IC₉₀ for ddC, determined in each assay run, should be between 0.1 and 0.3 µg/mL. Finally, the plateau level of viral RNA produced by an effective protease inhibitor should be less than 10% of the level achieved in an uninhibited infection. A compound was considered active if its IC₉₀ was found to be less than 1µM.

For antiviral potency tests, all manipulations in microtiter plates, following the initial addition of 2X concentrated compound solution to a single row of wells, were performed using a Perkin Elmer/Cetus ProPette.

### Dosage and Formulation

The antiviral compounds of this invention can be administered as treatment for viral infections by any means that produces contact of the active agent with the agent's site of action, i.e., the viral protease, in the body of a mammal. They can be administered by any conventional means available for use in conjunction with pharmaceuticals, either as individual therapeutic agents or in a combination of therapeutic agents. They can be administered alone, but preferably are administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to about 1000 milligrams per kilogram of body weight, with the preferred dose being about 0.1 to about 30 mg/kg.

Dosage forms of compositions suitable for administration contain from about 1 mg to about 100 mg of active ingredient per unit. In these pharmaceutical compositions the active ingredient will ordinarily be present in an amount of about 0.5-95% by weight based on the total weight of the composition. The active ingredient can be administered orally in solid dosage forms, such as capsules, tablets and powders, or in liquid dosage forms, such as elixirs, syrups and suspensions. It can also be administered parenterally, in sterile liquid dosage forms.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract. Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts, and sodium EDTA. In addition, parenteral solutions can contain preservatives, such as benzalkonium chloride, methyl- or propyl-paraben and chlorobutanol. Suitable pharmaceutical carriers are described in *Remington's Pharmaceutical Sciences, supra,* a standard reference text in this field.

Useful pharmaceutical dosage-forms for administration of the compounds of this invention can be illustrated as follows:

### Capsules

A large number of unit capsules can be prepared by filling standard two-piece hard gelatin capsules each with 100 mg of powdered active ingredient, 150 mg of lactose, 50 mg of cellulose, and 6 mg magnesium stearic.

### Soft Gelatin Capsules

A mixture of active ingredient in a digestible oil such as soybean oil, cottonseed oil or olive oil can be prepared and injected by means of a positive displacement pump into gelatin to form soft gelatin capsules containing 100 mg of the active ingredient. The capsules should then be washed and dried.

### Tablets

A large number of tablets can be prepared by conventional procedures so that the dosage unit is 100 mg of active ingredient, 0.2 mg of colloidal silicon dioxide, 5 milligrams of magnesium stearate, 275 mg of microcrystalline cellulose, 11 mg of starch and 98.8 mg of lactose. Appropriate coatings may be applied to increase palatability or delay absorption.

### Suspension

An aqueous suspension can be prepared for oral administration so that each 5 ml contain 25 mg of finely divided active ingredient, 200 mg of sodium carboxymethyl cellulose, 5 mg of sodium benzoate, 1.0 g of sorbitol solution, U.S.P., and 0.025 mg of vanillin.

### Injectable

A parenteral composition suitable for administration by injection can be prepared by stirring 1.5% by weight of active ingredient in 10% by volume propylene glycol and water. The solution is sterilized by commonly used techniques.

### Combination of components (a) and (b)

Each therapeutic agent component of this invention can independently be in any dosage form, such as those described above, and can also be administered in various ways, as described above. In the following description component (b) is to be understood to represent one or more agents as described previously. Thus, if components (a) and (b) are to be treated the same or independently, each agent of component (b) may also be treated the same or independently.

Components (a) and (b) of the present invention may be formulated together, in a single dosage unit (that is, combined together in one capsule, tablet, powder, liquid, etc.) as a combination product. When component (a) and (b) are not formulated together in a single dosage unit, component (a) may be administered at the same time as component (b) or in any order; for example component (a) of this invention may be administered first, followed by administration of component (b), or they may be administered in the reverse order. If component (b) contains more that one agent, e.g., one RT inhibitor and one protease inhibitor, these agents may be administered together or in any order. When not administered at the same time, preferably the administration of component (a) and (b) occurs less than about one hour apart. Preferably, the route of administration of component (a) and (b) is oral. The terms oral agent, oral inhibitor, oral compound, or the like, as used herein, denote compounds which may be orally administered. Although it is preferable that component (a) and component (b) both be administered by the same route (that is, for example, both orally) or dosage form, if desired, they may each be administered by different routes (that is, for example, one component of the combination product may be administered orally, and another component may be administered intravenously) or dosage forms.

As is appreciated by a medical practitioner skilled in the art, the dosage of the combination therapy of the invention may vary depending upon various factors such as the pharmacodynamic characteristics of the particular agent and its mode and route of administration, the age, health and weight of the recipient, the nature and extent of the symptoms, the kind of concurrent treatment, the frequency of treatment, and the effect desired, as described above.

The proper dosage of components (a) and (b) of the present invention will be readily ascertainable by a medical practitioner skilled in the art, based upon the present disclosure. By way of general guidance, typically a daily dosage may be about 100 milligrams to about 1.5 grams of each component. If component (b) represents more than one compound, then typically a daily dosage may be about 100 milligrams to about 1.5 grams of each agent of component (b). By way of general guidance, when the compounds of component (a) and component (b) are administered in combination, the dosage amount of each component may be reduced by about 70-80% relative to the usual dosage of the component when it is administered alone as a single agent for the treatment of HIV infection.

The combination products of this invention may be formulated such that, although the active ingredients are combined in a single dosage unit, the physical contact between the active ingredients is minimized. In order to minimize contact, for example, where the product is orally administered, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines.

Another embodiment of this invention where oral administration is desired provides for a combination product wherein one of the active ingredients is coated with a sustained-release material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose or other appropriate materials as known in the art, in order to further separate the active components. The polymer coating serves to form an additional barrier to interaction with the other component. In each formulation wherein contact is prevented between components (a) and (b) via a coating or some other material, contact may also be prevented between the individual agents of component (b).

Dosage forms of the combination products of the present invention wherein one active ingredient is enteric coated can be in the form of tablets such that the enteric coated component and the other active ingredient are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present invention can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

These as well as other ways of minimizing contact between the components of combination products of the present invention, whether administered in a single dosage form or administered in separate forms but at the same time or concurrently by the same manner, will be readily apparent to those skilled in the art, based on the present disclosure.

Pharmaceutical kits useful for the treatment of HIV infection, which comprise a therapeutically effective amount of a pharmaceutical composition comprising a compound of component (a) and one or more compounds of component (b), in one or more sterile containers, are also within the ambit of the present invention. Sterilization of the container may be carried out using conventional sterilization methodology well known to those skilled in the art. Component (a) and component (b) may be in the same sterile container or in separate sterile containers. The sterile containers of materials may comprise separate containers, or one or more multi-part containers, as desired. Component (a) and component (b), may be separate, or physically combined into a single dosage form or unit as described above. Such kits may further include, if desired, one or more of various conventional pharmaceutical kit components, such as for example, one or more pharmaceutically acceptable carriers, additional vials for mixing the components, etc., as will be readily apparent to those skilled in the art. Instructions, either as inserts or as labels, indicating quantities of the components to be administered, guidelines for administration, and/or guidelines for mixing the components, may also be included in the kit.

## Claims

1. A compound of formula II: 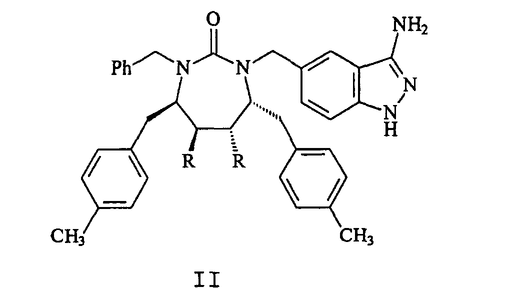 wherein each R is OH or a pharmaceutically acceptable salt thereof,
or a compound of formula II wherein both R groups taken together form a moiety selected from the group -O-, -OCH₂SCH₂O-, -OC(=O)O-, -OCH₂O-, -OC(=S)O-, -OC(=O)C(=O)O-, -OC(CH₃)₂O-, -OC((CH₂)₃NH₂)(CH₃)O-, -OC(OCH₃)(CH₂CH₂CH₃)O-, or -OS(=O)O-.

2. A pharmaceutical composition comprising a pharmaceutically acceptable carrier and a therapeutically effective amount of a compound of claim 1.

3. A compound of claim 1 for use in treating HIV infection.

4. A combination of a compound of claim 1 and at least one compound selected from the group consisting of HIV reverse transcriptase inhibitors and HIV protease inhibitors, for use in treating HIV infection.

5. A combination according to claim 4, wherein the reverse transcriptase inhibitor is a nucleoside reverse transcriptase inhibitor.

6. A combination according to claim 5, wherein the nucleoside reverse transcriptase inhibitor is selected from AZT, 3TC, ddI, ddC, and d4T and the protease inhibitor is selected from saquinavir, ritonavir, indinavir, VX-478, nelfinavir, KNI-272, CGP-61755, and U-103017.

7. A combination according to claim 6, wherein the nucleoside reverse transcriptase inhibitor is selected from AZT and 3TC and the protease inhibitor is selected from saquinavir, ritonavir, and indinavir.

8. A combination according to claim 7, wherein the nucleoside reverse transcriptase inhibitor is AZT.

9. A combination according to claim 7, wherein the protease inhibitor is indinavir.

10. A pharmaceutical kit useful for the treatment of HIV infection, which comprises a therapeutically effective amount of:
(a) a compound of claim 1; and
(b) at least one compound selected from the group consisting of HIV reverse transcriptase inhibitors and HIV protease inhibitors, in one or more sterile containers.

11. Use of a compound according to claim 1 in the acceptable salt thereof in the manufacture of a medicament for the treatment of HIV infection.

## Patentansprüche

1. Verbindung der Formel II: 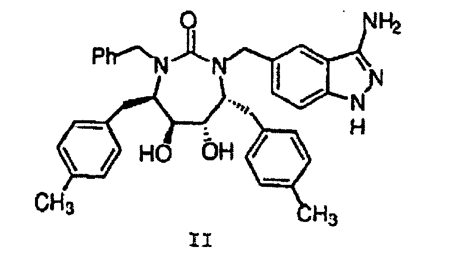 worin jedes R OH ist oder ein pharmazeutisch zulässiges Salz davon,
oder eine Verbindung der Formel II, worin beide R-Gruppen zusammengenommen einen Teil bilden, ausgewählt aus der Gruppe:
-O-, -OCH₂SCH₂O-, -OC(=O)O-, -OCH₂O-, -OC(=S)O-, -OC(=O)C(=O)O-, -OC(CH₃)₂O-, -OC((CH₂)₃NH₂)(CH₃)O-, -OC(OCH₃)(CH₂CH₂CH₃)O- oder -OS(=O)O-.

2. Pharmazeutische Zusammensetzung, aufweisend einen pharmazeutisch zulässigen Träger und eine therapeutisch wirksame Menge einer Verbindung nach Anspruch 1.

3. Verbindung nach Anspruch 1 zur Verwendung bei der Behandlung der HIV-Infektion.

4. Kombination einer Verbindung nach Anspruch 1 und mindestens einer Verbindung, ausgewählt aus der Gruppe, bestehend aus HIV-Revertase-Inhibitoren und HIV-Protease-Inhibitoren zur Verwendung bei der Behandlung von HIV-Infektion.

5. Kombination nach Anspruch 4, bei welcher der Revertase-Inhibitor ein Nukleosid-Revertase-Inhibitor ist.

6. Kombination nach Anspruch 5, bei welcher der Nukleosid-Revertase-Inhibitor ausgewählt ist aus: AZT, 3TC, ddI, ddC und d4T und der Protease-Inhibitor ausgewählt ist aus: Saquinavir, Ritonavir, Indinavir, VX-478, Nelfinavir, KNI-272, CGP-61755 und U-103017.

7. Kombination nach Anspruch 6, bei welcher der Nukleosid-Revertase-Inhibitor ausgewählt ist aus AZT und 3TC und der Protease-Inhibitor ausgewählt ist aus Saquinavir, Ritonavir und Indinavir.

8. Kombination nach Anspruch 7, bei welcher der Nukleosid-Revertase-Inhibitor AZT ist.

9. Kombination nach Anspruch 7, bei welcher der Protease-Inhibitor Indinavir ist.

10. Pharmazeutischer Satz, verwendbar zur Behandlung von HIV-Infektion, der eine therapeutisch wirksame Menge aufweist von:
(a) eine Verbindung nach Anspruch 1; und
(b) mindestens eine Verbindung, ausgewählt aus der Gruppe, bestehend aus HIV-Revertase-Inhibitoren und HIV-Protease-Inhibitoren in einem oder mehreren sterilen Behältern.

11. Verwendung einer Verbindung nach Anspruch 1 in dem zulässigen Salz davon für die Herstellung eines Medikaments zur Behandlung von HIV-Infektion.

## Revendications

1. Composé de la formule II: 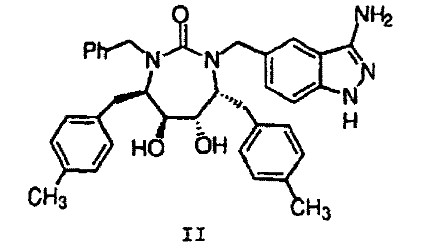 dans laquelle chaque R est OH ou sel pharmaceutiquement acceptable de celui-ci,
ou un composé de la formule II dans laquelle les deux groupes R pris ensemble forment une fraction choisie dans le groupe -O-, -OCH₂SCH₂O-, -OC(=O)O-, -OCH₂O-, -OC(=S)O-, -OC(=O)C(=O)O-, -OC(CH₃)₂O-, -OC((CH₂)₃NH₂)(CH₃)O-, -OC(OCH₃)(CH₂CH₂CH₃)O- ou -OS(=O)O-.

2. Composition pharmaceutique comprenant un excipient pharmaceutiquement acceptable et une quantité thérapeutiquement efficace d'un composé suivant la revendication 1.

3. Composé suivant la revendication 1, pour une utilisation dans le traitement d'une infection de VIH.

4. Combinaison d'un composé suivant la revendication 1 et d'au moins un composé choisi dans le groupe constitué d'inhibiteurs de transcriptase inverse de VIH et d'inhibiteurs de protéase de VIH, pour une utilisation dans le traitement d'une infection de VIH.

5. Combinaison suivant la revendication 4, dans laquelle l'inhibiteur de transcriptase inverse est un inhibiteur de transcriptase inverse nucléoside.

6. Combinaison suivant la revendication 5, dans laquelle l'inhibiteur de transcriptase inverse nucléoside est choisi parmi l'AZT, la 3TC, la ddI, la ddC et la d4T et l'inhibiteur de protéase est choisi parmi le saquinavir, le ritonavir, l'indinavir, le VX-478, le nelfinavir, le KNI-272, le CGP-61755 et le U-103017.

7. Combinaison suivant la revendication 6, dans laquelle l'inhibiteur de transcriptase inverse nucléoside est choisi parmi l'AZT et la 3TC et l'inhibiteur de protéase est choisi parmi le saquinavir, le ritonavir et l'indinavir.

8. Combinaison suivant la revendication 7, dans laquelle l'inhibiteur de transcriptase inverse nucléoside est l'AZT.

9. Combinaison suivant la revendication 7, dans laquelle l'inhibiteur de protéase est l'indinavir.

10. Kit pharmaceutique utile pour le traitement d'une infection de VIH, qui comprend une quantité thérapeutiquement efficace:
(a) d'un composé suivant la revendication 1; et
(b) d'au moins un composé choisi dans le groupe constitué d'inhibiteurs de transcriptase inverse de VIH et d'inhibiteurs de protéase de VIH, dans un ou plusieurs récipients stériles.

11. Utilisation d'un composé suivant la revendication 1 dans le sel acceptable de celui-ci dans la fabrication d'un médicament pour le traitement d'une infection de VIH.
